# FASCICULE DE BREVET EUROPEEN

(11) **EP 2 688 462 B1**
(45) Date de publication et mention de la délivrance du brevet: **31.08.2016**
(21) Numéro de dépôt: 12710772.0
(22) Date de dépôt: 08.03.2012
(51) Int. Cl.: A61B 3/113

(54) **DISPOSITIF ET PROCÉDÉ DE DÉTERMINATION DE L' OEIL DIRECTEUR D'UN PATIENT**
VORRICHTUNG UND VERFAHREN ZUR BESTIMMUNG DES DOMINANTEN AUGES BEI EINEM PATIENTEN
DEVICE AND PROCESS FOR DETERMINING THE DOMINANT EYE OF A PATIENT

(30) Priorité: 25.03.2011 FR 1100896
(43) Date de publication de la demande: 29.01.2014
(73) Titulaire: Essilor International (Compagnie Générale d'Optique), 94220 Charenton-le-Pont (FR)
(72) Inventeur: BONNIN, Thierry, 94220 Charenton Le Pont (FR); DIVO, Fabien, 94220 Charenton Le Pont (FR); PETIGNAUD, Cécile, 94220 Charenton Le Pont (FR); POULAIN, Isabelle, 94220 Charenton Le Pont (FR); PINAULT, Philippe, 94220 Charenton Le Pont (FR); ROUSSEL, Laurent, 94220 Charenton Le Pont (FR)
(74) Mandataire: Chauvin, Vincent
(86) Numéro de dépôt international: PCT/FR2012/000083
(87) Numéro de publication internationale: WO 2012/131182

(56) Documents cités:
- EP-A2- 0 590 231
- WO-A1-89/02243
- WO-A1-2011/021936
- US-A1- 2010 245 768
- O.SEIJAS ET AL.,: "Ocular dominance diagnosis and its influence in monovision", AMERICAN JOURNAL OF OPHTHALMOLOGY, vol. 144, no. 2, 2007, pages 209-216,216.E1, XP002651248,

## Description

### DOMAINE TECHNIQUE AUQUEL SE RAPPORTE L'INVENTION

La présente invention concerne de manière générale le domaine de la lunetterie.

Elle concerne plus particulièrement un dispositif et un procédé de mesure pour l'acquisition de l'oeil directeur d'un patient.

### ARRIERE-PLAN TECHNOLOGIQUE

La fabrication d'une paire de lunettes se décompose en six opérations principales :
- l'acquisition de paramètres relatifs au patient,
- le calcul des formes des faces optiques des lentilles, en fonction de ces paramètres acquis,
- le moulage et l'usinage des faces optiques des lentilles,
- l'acquisition de données relatives à la monture de lunettes sélectionnée par le patient, dont en particulier les formes des contours des entourages de cette monture,
- le centrage des lentilles ophtalmiques, qui consiste à positionner convenablement les contours des entourages sur chaque lentille, de manière qu'une fois usinées suivant ces contours puis montées sur la monture, ces lentilles exercent au mieux les fonctions optiques pour lesquelles elles ont été conçues, puis
- le détourage des lentilles.

On recherche actuellement, dans un souci d'améliorer le confort visuel du patient, à optimiser les formes et performances optiques des lentilles, notamment celles des lentilles à variation progressive de puissances (communément appelées « lentilles progressives »), et à améliorer la qualité de leur centrage dans les entourages de la monture de lunettes.

Il est pour cela nécessaire de considérer un nombre croissant de paramètres relatifs au patient.

Parmi ces paramètres, on cherche désormais à déterminer l'oeil directeur du patient afin notamment de calculer et d'usiner de manière personnalisée les lentilles du patient.

Pour déterminer l'oeil directeur du patient, on connaît différentes méthodes empiriques qui s'avèrent en pratique peu sûres, puisqu'entièrement fondées sur l'habileté et l'aisance du patient à les mettre en oeuvre.

Une méthode très répandue est l'épreuve du « trou dans la carte » appelée également « Hold in the Card Test » ou méthode de Dolman.

Cette méthode s'avère l'une des plus fiables pour identifier l'oeil directeur d'un individu. Cette méthode, telle qu'elle est par exemple décrite dans le document O.SEIJAS ET AL. : "Ocular dominance diagnosis and its influence in monovision", AMERICAN JOURNAL OF OPHTHALMOLOGY, vol. 144, no. 2, 2007, pages 209-216,216.EI, XP002651248, consiste à :
- donner au patient une carte avec un trou en son centre,
- demander au patient de tenir cette carte à deux mains, à bout de bras, puis à
- demander au patient de conserver ses deux yeux ouverts et de viser une cible se trouvant à distance devant lui à travers le trou (en position de visée, le sujet perçoit la cible centrée dans le trou).

Le patient ferme ensuite alternativement chacun de ses deux yeux de façon à prendre conscience de son oeil directeur qui, en pratique, est l'oeil aligné avec la cible et le trou. Ainsi, si la cible est toujours centrée dans le trou lorsqu'il ferme son oeil gauche, son oeil droit est directeur. Inversement, si la cible est toujours centrée dans le trou lorsqu'il ferme son oeil droit, son oeil gauche est directeur.

La détermination de l'oeil directeur par cette méthode est donc subjective puisqu'elle est le résultat du retour verbal du patient concernant sa perception de la cible.

### OBJET DE L'INVENTION

Afin de remédier à l'inconvénient d'un résultat basé sur une réponse subjective du patient, la présente invention propose un dispositif et un procédé de mesure objective permettant de déterminer l'oeil directeur du patient, qui est au moins en partie automatisé pour réduire le risque d'erreur lié à une mesure subjective.

Plus particulièrement, on propose selon l'invention un dispositif de détermination de l'oeil directeur d'un patient tel que défini dans la revendication 1.

On propose également selon l'invention un procédé de détermination de l'oeil directeur d'un patient tel que défini dans la revendication 10

Le principe ici utilisé pour déterminer l'oeil directeur du patient consiste à laisser le patient viser naturellement la cible au travers de la fenêtre de visée du masque, à l'aide de l'un ou l'autre de ses deux yeux, en gardant de préférence ses deux yeux ouverts. L'oeil naturellement utilisé pour la visée est en pratique l'oeil directeur du patient.

L'automatisation de la méthode consiste ensuite à acquérir une image du patient lorsqu'il vise cette cible.

En effet, une fois la cible correctement visée par le patient et alors qu'au moins un de ses yeux est encore caché par la paroi d'obturation du masque, le moyen d'acquisition peut, grâce aux propriétés de transmission de la lumière de la paroi d'obturation, acquérir une image sur laquelle apparaissent le masque et les deux yeux du patient.

En repérant sur cette image la position de la fenêtre de visée du masque par rapport aux deux yeux du patient, il est ainsi possible d'en déduire lequel des deux yeux du patient est son oeil directeur.

On observe alors que, puisque cette étape de déduction n'est pas réalisée par le patient lui-même, ce dernier adopte un comportement plus naturel pour viser la cible et peut réaliser la tâche de visée en gardant constamment les deux yeux ouverts, condition idéale pour mettre ses deux yeux en réelle concurrence.

D'autres caractéristiques avantageuses et non limitatives du dispositif conforme à l'invention sont définies dans les revendications 2 à 9.

D'autres caractéristiques avantageuses et non limitatives du procédé conforme à l'invention sont définies dans les revendications 11 à 18.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue schématique de côté d'un dispositif de détermination de l'oeil directeur d'un patient selon l'invention,
- la figure 2 est une vue schématique du visage du patient,
- la figure 3 est une vue schématique de dessus du dispositif de la figure 1, et
- les figures 4 et 5 sont des vues schématiques de deux variantes de réalisation du masque du dispositif de la figure 1.

En préliminaire, on notera que les éléments identiques ou similaires des différentes variantes de réalisation de l'invention représentées sur les différentes figures seront, dans la mesure du possible, référencés par les mêmes signes de référence et ne seront pas décrits à chaque fois.

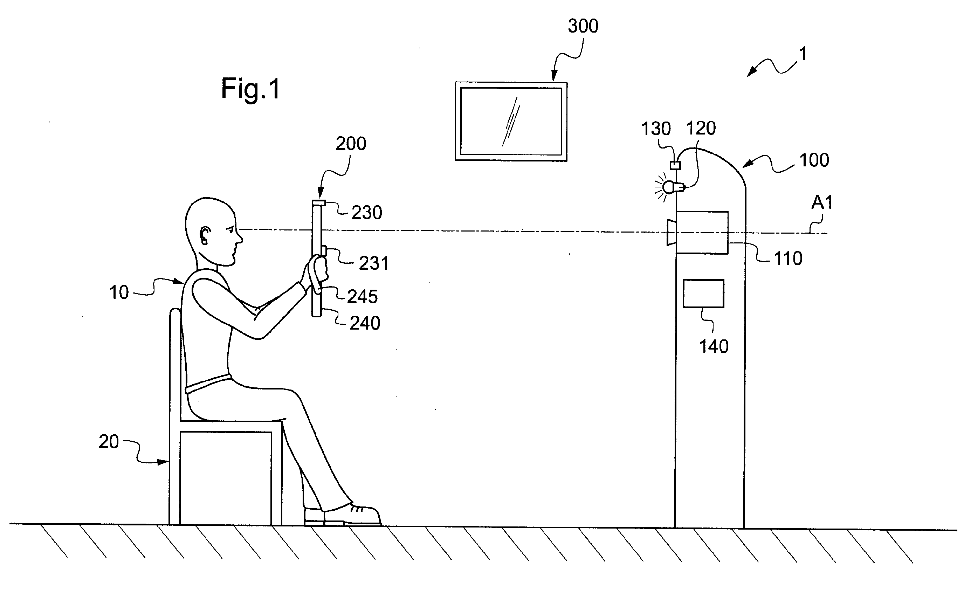

Sur la figure 1, on a représenté un dispositif de détermination 1 de l'oeil directeur d'un patient 10.

Ce dispositif de détermination 1 comporte trois éléments principaux, à savoir une cible 120 visible par le patient 10, un moyen d'acquisition 110 d'une image du visage du patient 10, et un masque 200 à interposer entre, d'un côté, le visage du patient 10, et, de l'autre, la cible 120 et le moyen d'acquisition 110.

On notera d'ores et déjà que la cible 120 et le moyen d'acquisition 110 pourraient être confondus en ce sens que le moyen d'acquisition, visible par le patient, pourrait constituer la cible à viser.

En effet, la cible 120 que le patient 10 doit viser peut être constituée par un objet, un motif, une lumière ou plus généralement par tout moyen susceptible d'être vu par le patient 10.

Telle que représentée sur la figure 1, la cible 120 est constituée par une source de lumière, en l'espèce une diode électroluminescente.

Cette cible 120 est ici située sur une colonne, appelée totem 100, disposée en face du siège 20 sur lequel le patient 10 s'assoit lors de la mesure.

Cette cible 120 est plus précisément située sensiblement à la même hauteur que les yeux 12 du patient 10, et à une distance de celui-ci comprise entre un mètre vingt et un mètre et demi.

Le moyen d'acquisition 110 est ici également situé sur le totem 100, sensiblement à la même hauteur que les yeux 12 du patient 10.

Il est pour cela ici situé en dessous de la cible 120, à proximité de celle-ci.

Ce moyen d'acquisition 110 peut être constitué par tout système adapté à acquérir une image du patient 10.

Tel que représenté sur la figure 1, ce moyen d'acquisition est une caméra 110 numérique d'axe optique A1. Il pourrait également être formé par un appareil photo numérique.

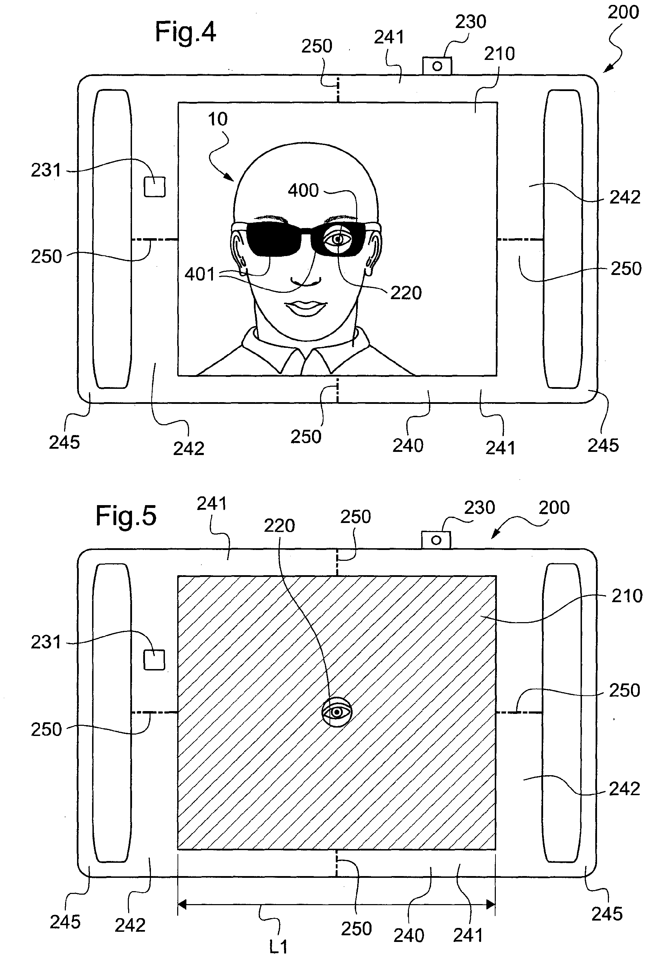

Comme le montre mieux la figure 5, le masque 200 comprend quant à lui au moins une paroi d'obturation 210 et une fenêtre de visée 220 située au travers de la paroi d'obturation 210.

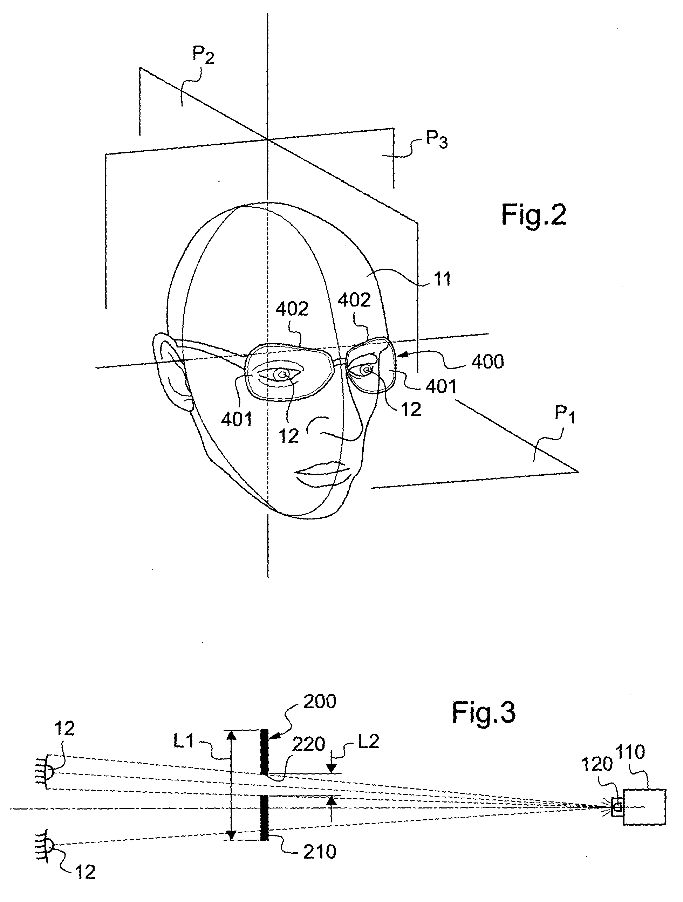

Comme le montre la figure 3, la fenêtre de visée 220 est conçue pour permettre au patient 10 de viser la cible 120 à l'aide de l'un de ses deux yeux 12 uniquement. On observe ici que l'oeil utilisé le plus naturellement pour la visée est en pratique l'oeil directeur du patient 10.

La paroi d'obturation 210 du masque 200 est quant à elle conçue pour permettre de cacher la cible 120 à l'autre oeil du patient 10. Elle est en outre avantageusement conçue de telle manière qu'elle permet au moyen d'acquisition 110 d'acquérir une image d'au moins une partie du visage du patient 10 lorsqu'il vise la cible 120 (cette partie du visage étant suffisante pour permettre de déduire de l'image acquise lequel des deux yeux 12 du patient est son oeil directeur).

En l'espèce, le masque 200 comporte également un châssis 240 en forme de cadre, qui entoure la paroi d'obturation 210 et qui permet au patient de manoeuvrer plus facilement le masque 200.

Comme le montre la figure 5, la paroi d'obturation 210 du masque 200 présente des dimensions telles qu'elle est adaptée à s'interposer entre la cible 120 et les deux yeux 12 du patient 10.

Elle est en particulier adaptée à cacher la cible 120 à l'un des yeux 12 du patient 10 lorsque le patient vise la cible 120 avec l'autre oeil au travers de la fenêtre de visée 220.

Dans l'exemple représenté sur les figures 3 et 5, cette paroi d'obturation présente une forme rectangulaire, de largeur L1 comprise entre 100 et 300 millimètres, et de hauteur comprise entre 100 et 300 millimètres.

Pour cacher la cible 120 à au moins l'un des deux yeux 12 du patient 10, cette paroi d'obturation 210 est prévue pour ne transmettre, au moins momentanément, qu'au maximum une partie de la lumière qui se propage depuis la cible 120 vers le patient 10.

Pour permettre à la caméra 110 d'acquérir une image du visage du patient 10, cette paroi d'obturation 210 est prévue pour transmettre, au moins momentanément, au moins une partie de la lumière qui se propage depuis le visage du patient 10 vers la caméra 110.

Des exemples détaillés de réalisation de cette paroi d'obturation 210 seront donnés dans la suite de cet exposé.

La fenêtre de visée 220, qui est prévue pour permettre au patient 10 de viser la cible 120, est située à distance des bords de la paroi d'obturation 210 de manière à s'assurer que, lorsque le patient 10 vise la cible 120 avec un oeil, son autre oeil reste caché derrière la paroi d'obturation 210 du masque 200.

La fenêtre de visée 220 est préférentiellement située à mi-largeur de la paroi d'obturation 210.

Elle présente des dimensions telles que la cible 120 n'est visible que par un seul des deux yeux 12 du patient 10 lorsque le patient 10 tient le masque 200 à bout de bras (figure 1).

Dans l'exemple représenté sur les figures, la fenêtre de visée 220 présente une forme de disque centré au milieu de la paroi d'obturation 210, de diamètre L2 compris entre 10 et 40 millimètres, préférentiellement égal à 25 millimètres.

Le châssis 240 en forme de cadre comporte quant à lui deux branches longitudinales 241 et deux branches latérales 242. Ses deux branches latérales 242 présentent des largeurs importantes et sont ouvertes par de longues ouvertures qui forment deux poignées 245 permettant au patient 10 de manoeuvrer le masque 200 avec aisance.

Le caractère portable du masque 200 permet ainsi au patient 10 d'ajuster la position de ce masque 200 manuellement, de telle manière qu'il puisse placer la fenêtre de visée 220 du masque 200 entre son oeil directeur et la cible 120.

Bien entendu, en variante, on pourrait prévoir que le masque 200 soit fixe, auquel cas le patient 10 serait forcé de mouvoir son visage afin de pouvoir viser la cible 120 au travers de la fenêtre de visée 220 du masque 200.

Dans un premier mode de réalisation du masque 200, la paroi d'obturation 210 et la fenêtre de visée 220 sont passives en ce sens qu'elles présentent des caractéristiques de transmission de la lumière invariables dans le temps.

Pour permettre à la paroi d'obturation 210 de cacher la cible 120 à au moins l'un des deux yeux 12 du patient 10 tout en permettant à la caméra 110 de pouvoir capter une image du visage du patient, il est prévu que la paroi d'obturation 210 présente des caractéristiques optiques spécifiques.

Telle que représentée sur la figure 4, la paroi d'obturation 210 est formée d'une plaque polarisante qui polarise la lumière dans un premier sens alors que la fenêtre de visée 220 n'est pas polarisée (ou est éventuellement polarisée perpendiculairement ou dans un sens inverse).

Pour que la paroi d'obturation 210 du masque 200 cache la cible 120 à au moins l'un des deux yeux du patient 10, il est alors prévu d'équiper ce dernier d'une paire de lunettes 400 comportant une monture équipée de deux entourages 402, et deux lentilles 401 qui sont montées dans les entourages 402 et qui sont polarisées dans un second sens (inverse ou perpendiculaire au premier sens).

Ainsi le patient 110 ne voit-il la cible 120 qu'au travers de la seule fenêtre de visée 220 du masque 200.

La polarisation de la paroi d'obturation 210 et des lentilles 401 peut être rectiligne. La paroi d'obturation 210 pourrait alors être polarisée verticalement tandis que les lentilles 401 seraient polarisées horizontalement (en position portée par le patient). La particularité de ce type de polarisation est que, lorsque le patient 10 incline sa tête ou le masque, les directions de polarisation des lentilles 401 et de la paroi d'obturation 210 ne sont plus parfaitement orthogonales, ce qui rend la paroi d'obturation 210 légèrement transparente pour le patient 10.

La polarisation de la paroi d'obturation 210 et des lentilles 401 peut également être circulaire. La paroi d'obturation 210 pourrait alors être polarisée dans le sens horaire tandis que les lentilles 401 seraient polarisées dans le sens trigonométrique. De cette manière, l'inclinaison de la tête du patient 10 n'influe pas sur l'opacité de la paroi d'obturation 210 vue par le patient 10.

Comme le montre la figure 4, en position de visée, le patient 10 ne peut voir la cible qu'avec un seul de ses deux yeux 12, tandis que la caméra 110 peut acquérir une image du visage du patient sur laquelle apparaissent les deux lentilles 401 de la paire de lunettes 400, ainsi que l'oeil directeur du patient 10.

En variante, on pourrait prévoir que la paire de lunettes 400 soit remplacée par un équipement comportant deux verres polarisés, et des moyens de fixation de ces deux verres polarisés sur une paire de lunettes classique équipant le patient.

Encore en variante, on pourrait prévoir que la paire de lunettes 400 soit remplacée par un équipement comportant une plaquette polarisée de forme par exemple rectangulaire, et des moyens de fixation de cette plaquette polarisée sur une paire de lunettes classique équipant le patient.

Selon une autre variante du masque 200 dans laquelle la paroi d'obturation 210 et la fenêtre de visée 220 sont passives, on pourrait prévoir que la paroi d'obturation 210 présente un taux de transmission de la lumière au moins trois fois supérieur dans un sens que dans l'autre.

Elle pourrait même présenter un taux de transmission de la lumière non nul dans un sens (depuis le patient vers la caméra) et nul dans l'autre (depuis la cible vers le patient). A titre d'exemple, la paroi d'obturation 210 pourrait ainsi être formée par un miroir semi-réfléchissant (ou « miroir sans tain »). Ainsi la caméra 110 pourrait-elle filmer le visage du patient 10 alors que la paroi d'obturation 110 cache la cible 120 à au moins l'un des deux yeux 12 du patient.

Dans un second mode de réalisation du masque 200, la paroi d'obturation 210 est active en ce sens qu'elle présente des caractéristiques de transmission de la lumière variables dans le temps.

Dans ce mode de réalisation, les caractéristiques optiques de la paroi d'obturation 210 sont pilotées entre un état obscurci, dans lequel la paroi d'obturation 210 cache la cible 120 à au moins un des deux yeux 12 du patient 10, et un état transparent, dans laquelle la paroi d'obturation 210 laisse le visage du patient visible à la caméra 110.

Telle que représentée sur la figure 5, la paroi d'obturation 210 est formée, avec la fenêtre de visée 220, par un écran à cristaux liquides.

La fenêtre de visée 220 est alors pilotée pour rester à l'état transparent en continu. L'opacité de la paroi d'obturation 210 est en revanche pilotée à l'état obscurci pendant que le patient tente de viser la cible 120, puis à l'état transparent lorsque le patient a la cible 120 en ligne de mire. Ainsi, en acquérant une image du visage du patient au moment même où la paroi d'obturation 210 passe à l'état transparent, on obtient un cliché sur lequel le patient 10 vise la cible 120.

Quel que soit le type actif ou passif de la paroi d'obturation 210, il est prévu des moyens de pilotage notamment pour piloter la caméra 110.

Tels que représentés sur la figure 1, ces moyens de pilotage comportent :
- une gâchette 231 facile d'accès pour le patient 10 puisque située sur le châssis 240 du masque 200, à côté de l'une des poignées 245,
- un émetteur-récepteur infra-rouge 230 situé sur le châssis 240 du masque 200, pour transmettre un signal relatif à la position enfoncée ou relâchée de la gâchette 231,
- un émetteur-récepteur infra-rouge 130 situé sur le totem 100, pour recevoir le signal relatif à la position enfoncée ou relâchée de la gâchette 231, et
- une unité informatique 140 pour piloter la caméra 110 en fonction de la position de la gâchette 231.

Si la paroi d'obturation 210 est du type inactif, l'unité informatique 140 est plus précisément adaptée à piloter la caméra 110 de telle manière que cette dernière acquiert une image du patient 10 dès que celui-ci enfonce la gâchette 231.

Si la paroi d'obturation 210 est du type actif, l'unité informatique 140 est adaptée à piloter la paroi d'obturation 210 de manière qu'elle passe de l'état obscurci à l'état transparent dès que le patient 10 enfonce la gâchette 231, puis à piloter la caméra 110 de telle manière que cette dernière acquiert une image du patient 10.

Préférentiellement, comme cela sera également exposé dans la suite de cet exposé, l'unité informatique 140 sera par ailleurs aussi adaptée à déduire de l'image acquise lequel des yeux 12 du patient 120 est son oeil directeur.

Le dispositif de détermination 1 représenté sur la figure 1 comporte enfin un écran d'affichage 300, tel qu'un écran LCD, adapté à afficher l'image du patient acquise par la caméra 110. Cet écran d'affichage 300 est alors piloté par l'unité informatique 140.

Il peut par ailleurs être éventuellement piloté pour afficher une image du visage du patient qui ait été préalablement traitée (par exemple éclaircie) et/ou pour superposer à cette image des informations supplémentaires telles que des indicateurs précisant les côtés « droit » et « gauche » de l'image ou précisant lequel des deux yeux 12 du patient 10 est son oeil directeur.

Le procédé pour déterminer l'oeil directeur du patient 10 à l'aide du dispositif de détermination 1 précité est alors mis en oeuvre de la manière suivante, en cinq étapes principales dont :
- une étape de positionnement du patient 10 et du masque 200 dans le champ de la caméra 110, de telle manière que la cible 120 soit cachée de l'un au moins des deux yeux 12 du patient 10 par la paroi d'obturation 210 du masque 200,
- une étape de visée de la cible 120 par le patient 10 au travers de la fenêtre de visée 220 du masque 200,
- une étape d'acquisition par la caméra 110 d'une image du visage du patient 10,
- une étape de déduction de l'oeil directeur du patient 10 en fonction de la position du masque 200 et du visage du patient 10 sur l'image acquise, et
- une étape d'affichage de l'image acquise sur l'écran d'affichage 300.

Au cours de la première étape de positionnement, l'opticien fait asseoir le patient 10 sur la chaise 20, en vis-à-vis du totem 100, et lui indique la position de la cible 120 afin qu'il l'identifie.

La position de la chaise 20 doit alors être ajustée de telle manière que le visage du patient 10 se trouve, dans l'idéal, positionné face à l'objectif de la caméra 110.

Cette position idéale peut être plus précisément définie en caractérisant le référentiel du visage du patient 10 au moyen de trois plans P₁, P₂, P₃ orthogonaux les uns aux autres.

Tel que représenté sur la figure 2, on peut ainsi définir un plan de Francfort P₁ passant par les points orbitaires inférieurs et le porion du patient (le porion étant le point du crâne le plus élevé du conduit auditif, qui correspond au tragion de l'oreille). Ce plan de Francfort P₁ doit alors idéalement être positionné sensiblement horizontalement, et passer par l'axe optique A1 de la caméra 110.

On peut également définir un plan sagittal P₂ comme étant le plan perpendiculaire au plan de Francfort P₁ et qui passe par la médiatrice des deux yeux 12 du patient 10 (la médiatrice étant l'axe qui passe au milieu du segment défini par les centres de rotation des deux yeux 12 et qui est parallèle au plan de Francfort P₁). Ce plan sagittal P₂ doit alors idéalement être positionné verticalement et passer par l'axe optique A1 de la caméra 110.

On peut enfin définir un plan frontal P₃ comme étant le plan qui est perpendiculaire au plan de Francfort P1 et au plan sagittal P₂ et qui passe par le sommet de la tête du patient 10. Ce plan frontal P₃ doit alors idéalement être positionné orthogonalement à l'axe optique A1 de la caméra 110.

L'image du visage du patient 10 peut bien entendu être acquise lorsque la tête du patient est légèrement inclinée ou décalée par rapport à cette position idéale.

Préférentiellement, l'image est toutefois acquise lorsque l'axe optique A1 de la caméra 110 fait un angle compris entre -20 et +20 degrés avec le plan frontal P₃ de la tête du patient 10, de manière à éviter toute erreur de mesure.

L'opticien demande ensuite au patient 10 de prendre le masque 200 par ses poignées 245, puis de le tenir à bout de bras afin de l'interposer entre son visage et la cible 120. Ainsi positionné, le masque 200 s'étend sur le chemin optique de la lumière émise par la cible 120 vers le patient.

La seconde étape de visée consiste, pour le patient 10, à déplacer le masque 200 dans une position dans laquelle il a la cible 120 en ligne de mire, au travers de la fenêtre de visée 220, en gardant de préférence les deux yeux ouverts.

Cette étape doit permettre au patient 10 de placer naturellement le masque 200 dans une position telle qu'il observe la cible 120 par son oeil directeur.

Le choix de l'un ou l'autre des deux yeux 12 du patient 10 pour observer la cible 120 par la fenêtre de visée 220 se fait en pratique naturellement avec l'oeil directeur.

Si le patient 10 hésite, l'opticien peut demander au patient 10 de rapprocher le masque 200 de son visage puis de l'en éloigner en gardant la cible en ligne de mire avec l'un puis avec l'autre de ses deux yeux 12. Le patient 10 aura alors généralement des difficultés à réaliser cette opération avec l'un de ses deux yeux 12 tandis qu'il effectuera cette opération sans difficulté avec l'autre de ses deux yeux 12, qui sera en pratique son oeil directeur.

La troisième étape d'acquisition d'une image du patient 10 est ici commandée par le patient lui-même, lorsqu'il a la cible 120 en ligne de mire.

Ainsi, lorsque le patient 10 appuie sur la gâchette 231, l'unité informatique 140 est programmée pour commander l'acquisition d'un cliché du visage du patient (après avoir éventuellement commandé la mise à l'état transparent de la paroi d'obturation 210).

En variante, on pourrait prévoir que l'opticien dispose d'une télécommande afin de commander lui-même cette acquisition d'image lorsqu'il estime que le patient 10 est en bonne posture.

Encore en variante, on pourrait prévoir que la caméra 110 acquiert en continu ou à intervalles réguliers des images du visage du patient 10, et que l'unité informatique 140 sélectionne l'une de ces images afin de déterminer sur cette image lequel des deux yeux 12 du patient 10 est son oeil directeur.

La sélection de cette image ne serait bien entendu pas aléatoire.

A titre d'exemple, on pourrait prévoir que la caméra 110 acquiert une image du visage du patient 10 toutes les secondes, puis que l'unité informatique 140 détermine, sur chacune de ces images, la position du visage du patient 10 par rapport au masque 200. L'unité informatique 140 pourrait alors sélectionner le cinquième des images consécutives sur lesquelles la position du visage du patient 10 par rapport au masque 200 est restée sensiblement stable. Autrement formulé, l'image sera sélectionnée lorsque le patient 10 aura gardé la cible 120 en ligne de mire avec le même oeil pendant une durée prédéterminée, ici égale à 5 secondes.

Dans cette variante, si l'écran est du type actif, on pourra notamment prévoir que la paroi d'obturation 210 passe de l'état obscurci à l'état transparent de manière régulière (ici toutes les secondes), durant une durée qui sera suffisamment réduite pour que le patient ne puisse pas percevoir ce changement d'état, mais qui sera suffisamment longue pour que la caméra 110 puisse acquérir une image nette du visage du patient 10.

Quelle que le soit la manière dont l'image sera acquise, cette image fera alors préférentiellement apparaître le masque 200 (en transparence) ainsi que les deux yeux 12 du patient 10 ou les deux entourages 402 de la paire de lunettes 400 portée par le patient 10 (voir figure 4).

La quatrième étape consiste alors à déduire, à partir de cette seule image, lequel des deux yeux 12 du patient 10 est son oeil directeur.

On pourra utiliser à cet effet une grande variété de techniques de traitement d'images, telles que l'une des quatre techniques suivantes.

La première technique n'est opérationnelle que lorsque l'image acquise fait apparaître les deux yeux 12 du patient 10.

Cette première technique consiste à calculer dans le plan de l'image, d'une part, les coordonnées des pupilles (ou des commissures) des deux yeux 12 du patient 10, et, d'autre part, les coordonnées du centre de la fenêtre de visée 220 du masque 200.

Alors, l'oeil directeur sera celui dont les coordonnées seront les plus proches de celles du centre de la fenêtre de visée 220.

La seconde technique, analogue à la première, n'est opérationnelle que lorsque l'image acquise fait apparaître les deux entourages 402 de la paire de lunettes 400 portée par le patient 10 (voir figure 4).

Cette seconde technique consiste alors à calculer, d'une part, les coordonnées du barycentre de chacun des deux entourages 402 de la paire de lunettes 400, et, d'autre part, les coordonnées du centre de la fenêtre de visée 220 du masque 200.

Alors, l'oeil directeur sera celui situé derrière l'entourage 402 dont les coordonnées du barycentre seront les plus proches de celles du centre de la fenêtre de visée 220.

La troisième technique est plus flexible, puisqu'elle est applicable que les deux yeux du patient 10 soient ou non visibles sur l'image acquise.

Cette technique sera préférentiellement utilisée lorsque l'image acquise sera particulièrement sombre et que les deux yeux 12 du patient 10 seront difficilement visibles sur cette image.

Cette troisième technique consiste à calculer, d'une part, les coordonnées du centre du masque 200 grâce à des motifs 250 dessinés sur le châssis 240 du masque 200 (ces coordonnées correspondant aux coordonnées du centre de la fenêtre de visée 220), et, d'autre part, les coordonnées du barycentre du contour du visage du patient 10. Ce contour est en effet plus visible que les yeux 12 du patient 10.

Alors, l'oeil directeur du patient sera son oeil gauche si le barycentre du contour du visage du patient 10 est décalé sur la gauche par rapport au centre du masque 200. Son oeil directeur sera au contraire son oeil droit si le barycentre du contour du visage du patient 10 est décalé sur la droite par rapport au centre du masque 200.

La quatrième technique est opérationnelle lorsque l'image acquise ne fait apparaître qu'un seul des deux yeux 12 du patient 10 (figure 4), à savoir son oeil directeur.

Cette quatrième technique consiste alors à calculer la forme de l'oeil du patient, à repérer la position droite ou gauche de la conjonctive bulbaire de l'oeil, et à en déduire si l'oeil directeur du patient 10 est respectivement son oeil gauche ou son oeil droit.

La cinquième étape d'affichage consiste, pour l'unité informatique 140, à commander l'affichage de l'image acquise sur l'écran d'affichage 300.

Cette image peut bien entendu être préalablement traitée, par exemple pour être au besoin éclaircie.

L'unité informatique 140 peut également commander l'affichage, en superposition de l'image acquise, d'indicateurs pour signaler lequel des côtés de l'image est le côté droit et le côté gauche (ce qui s'avère particulièrement utile si l'image affichée est une image vue en miroir).

L'unité informatique 140 peut également commander l'affichage, à côté de l'image acquise, d'un message indiquant lequel des deux yeux 12 du patient 10 est son oeil directeur.

Ainsi, grâce à cette étape d'affichage, le patient 10 pourra facilement se laisser convaincre duquel de ses deux yeux 12 est son oeil directeur.

La présente invention n'est nullement limitée aux modes de réalisation décrits et représentés, mais l'homme du métier saura y apporter toute variante conforme à son esprit.

En particulier, on pourra prévoir que l'étape de déduction de l'oeil directeur du patient soit réalisée par l'opticien ou par le patient lui-même, et non pas par l'unité informatique.

Cette étape de déduction, confondue avec l'étape d'affichage, consistera alors simplement pour l'unité informatique 140 à afficher, d'une part, l'image acquise sur l'écran d'affichage 300, et éventuellement, d'autre part, des indicateurs des côtés droit et gauche de l'image. Ainsi l'opticien et le patient pourront-ils facilement en déduire lequel des deux yeux 12 du patient 10 est son oeil directeur.

Selon une autre variante de l'invention, on pourra prévoir que le contour de l'objectif de la caméra 110 soit peint en rouge et qu'il forme ainsi la cible. De cette manière, lorsqu'il visera la cible 120, le patient 10 dirigera son regard dans l'axe optique de la caméra 110.

Selon une autre variante de l'invention, on pourra prévoir que, lors des étapes précitées de positionnement, de visée et d'acquisition, le patient 10 se tienne non pas assis mais debout.

Selon une autre variante de l'invention, on pourra prévoir que la cible 120 soit située, non pas dans le plan de l'objectif de la caméra 110, mais en avant ou en arrière de ce plan, sans que cette position n'influe sur la mise en oeuvre du procédé décrit.

Selon une variante du masque 200 dans lequel la paroi d'obturation 210 est active, on pourra prévoir que le masque comporte une plaque transparente recouverte, excepté au niveau de la fenêtre de visée 220, d'une couche optiquement activable, adaptée à être pilotée entre un état obscurci et un état transparent.

## Revendications

1. Dispositif de détermination de l'oeil directeur d'un patient (10), **caractérisé en ce qu'**il comporte :
- une cible (120) visible par le patient (10) en position de mesure,
- un moyen d'acquisition (110) d'une image du visage du patient (10) en position de mesure, et
- un masque (200) à positionner entre, d'un côté, le visage du patient (10) en position de mesure, et, de l'autre, la cible (120) et le moyen d'acquisition (110), comprenant :
a) une paroi d'obturation (210) :
a1) qui présente des dimensions telles qu'elle est adaptée à s'interposer entre la cible (120) et les deux yeux du patient (10) en position de mesure,
a2) adaptée à transmettre au maximum une partie de la lumière qui se propage depuis la cible (120) vers le patient (10), pour cacher la cible (120) au patient (10) en position de mesure, et
a3) adaptée à transmettre au moins une partie de la lumière qui se propage depuis le patient (10) vers le moyen d'acquisition (110), pour permettre au moyen d'acquisition (110) d'acquérir une image du visage du patient (10) en position de mesure, étant suffisante pour pour permettre de déduire de l'image acquise lequel des deux yeux du patient est son oeil directeur et
b) une fenêtre de visée (220) de la cible (120) par le patient (10) :
b1) qui est située au travers de la paroi d'obturation (210),
b2) adaptée à transmettre la lumière qui se propage depuis la cible (120) vers le patient (10), pour permettre au patient (10) de viser la cible (120), et
b3) qui présente des dimensions telles que la cible (120) n'est visible que par un seul oeil du patient (10) en position de mesure.

2. Dispositif selon la revendication 1, dans lequel la paroi d'obturation (210) et la fenêtre de visée (220) sont passives en ce sens qu'elles présentent des caractéristiques de transmission de la lumière invariables dans le temps.

3. Dispositif selon la revendication 2, dans lequel la paroi d'obturation (210) polarise la lumière linéairement ou circulairement, et dans lequel il est prévu une paire de lunettes (400) ou équivalent pour équiper le patient (10) en position de mesure, qui comporte des verres (401) polarisés respectivement linéairement ou circulairement, les verres (401) et la paroi d'obturation (210) étant polarisés rectilignement dans des directions orthogonales ou circulairement dans des sens inverses.

4. Dispositif selon la revendication 1, dans lequel la paroi d'obturation (210) est active en ce sens qu'elle présente des caractéristiques de transmission de la lumière variables.

5. Dispositif selon la revendication 4, dans lequel la paroi d'obturation (210) et la fenêtre de visée (220) forment ensemble un écran à cristaux liquides.

6. Dispositif selon la revendication 4, dans lequel la fenêtre de visée (220) est inactive en ce sens qu'elle présente des caractéristiques de transmission de la lumière invariables et dans lequel la paroi d'obturation (210) comporte une couche activable pilotée pour présenter une transparence ou une opacité complète.

7. Dispositif selon l'une des revendications précédentes, dans lequel il est prévu un écran d'affichage (300), adapté à afficher une image du patient (10) en position de mesure acquise par le moyen d'acquisition (110).

8. Dispositif selon l'une des revendications précédentes, dans lequel il est prévu une unité informatique (140) adaptée à traiter une image du patient (10) en position de mesure acquise par le moyen d'acquisition (110) et à en déduire l'oeil directeur du patient (10).

9. Dispositif selon l'une des revendications précédentes, dans lequel le masque (200) comporte un châssis (240) équipé d'au moins une poignée (245) de manoeuvre, et est portable par le patient (10).

10. Procédé de détermination de l'oeil directeur d'un patient (10) à l'aide d'un dispositif comportant une cible (120), un moyen d'acquisition (110) d'images et un masque (200) présentant une paroi d'obturation (210) ouverte par une fenêtre de visée (220), **caractérisé en ce qu'**il comporte :
- une étape de positionnement du patient (10) et du masque (200) dans le champ du moyen d'acquisition (110) d'images, de telle manière que le masque (200) soit situé entre la cible (120) et les deux yeux du patient (10),
- une étape de visée de la cible (120) par le patient (10), au travers de la fenêtre de visée (220),
- une étape d'acquisition par le moyen d'acquisition (110) d'une image du patient (10) visant la cible (120), sur laquelle apparaissent une partie au moins du visage du patient (10) suffisante pour permettre de déduire de l'image acquise lequel des deux yeux du patient est son oeil directeur et du masque (200), et
- une étape de déduction de l'oeil directeur du patient (10) en fonction de la position du masque (200) par rapport au visage du patient (10) sur l'image acquise.

11. Procédé selon la revendication 10, dans lequel l'étape de déduction consiste à afficher l'image acquise sur un écran d'affichage (300).

12. Procédé selon la revendication 10, dans lequel à l'étape de déduction, l'image acquise est traitée afin de calculer, d'une part, les positions des deux yeux du patient (10), et, d'autre part, la position de la fenêtre de visée (220) par rapport aux deux yeux du patient (10).

13. Procédé selon la revendication 10, dans lequel, le patient (10) étant équipé d'une paire de lunettes (400), à l'étape de déduction, l'image acquise est traitée afin de calculer, d'une part, les positions des deux entourages (402) de la paire de lunettes (400), et, d'autre part, la position de la fenêtre de visée (220) par rapport aux deux entourages (402).

14. Procédé selon la revendication 10, dans lequel, le masque (200) présentant au moins un repère visible (250), l'image acquise est traitée afin de calculer la position du visage du patient (10) par rapport au repère visible (250).

15. Procédé selon l'une des revendications 10 à 14, dans lequel l'acquisition d'image est commandée manuellement.

16. Procédé selon l'une des revendications 10 à 14, dans lequel l'acquisition d'image est réalisée en continu ou à intervalles réguliers et dans lequel, à l'étape de déduction, il est prévu de sélectionner l'une desdites images acquises.

17. Procédé selon la revendication 14, dans lequel l'image est sélectionnée lorsque la position du visage du patient (10) relativement au masque (200) est stabilisée.

18. Procédé selon l'une des revendications 10 et 17, dans lequel il est prévu une étape d'affichage de l'image acquise.

## Patentansprüche

1. Vorrichtung zum Bestimmen des führenden Auges eines Patienten (10), **dadurch gekennzeichnet, dass** sie Folgendes umfasst:
- ein Ziel (120), das für den Patienten (10) in der Messposition sichtbar ist,
- ein Mittel (110) zum Erfassen eines Bildes des Gesichts des Patienten (10) in der Messposition und
- eine Maske (200), die einerseits zwischen dem Gesicht des Patienten (10) in der Messposition und andererseits dem Ziel (120) und dem Erfassungsmittel (110) zu positionieren ist und Folgendes umfasst:
a) eine Abschirmwand (210),
a1) die Abmessungen besitzt, derart, dass sie zwischen das Ziel (120) und die beiden Augen des Patienten (10) in der Messposition eingefügt werden kann,
a2) die höchstens einen Teil des Lichts, das sich von dem Ziel (120) zu dem Patienten (10) ausbreitet, durchlassen kann, um das Ziel (120) für den Patienten (10) in der Messposition zu verbergen, und
a3) wenigstens einen Teil des Lichts, das sich von dem Patienten (10) zu dem Erfassungsmittel (110) ausbreitet, durchlassen kann, um dem Erfassungsmittel (110) zu ermöglichen, ein Bild des Gesichts des Patienten (10) in der Messposition zu erfassen, wobei der Teil ausreicht, um zu ermöglichen, aus dem erfassten Bild abzuleiten, welches der beiden Augen des Patienten sein führendes Auge ist, und
b) ein Fenster (220) zum Anvisieren des Ziels (120) durch den Patienten (10),
b1) das sich in der Abschirmwand (210) befindet,
b2) das das Licht, das sich von dem Ziel (120) zu dem Patienten (10) ausbreitet, durchlassen kann, um dem Patienten (10) zu ermöglichen, das Ziel (120) anzuvisieren, und
b3) das Abmessungen aufweist, derart, dass das Ziel (120) nur durch ein einziges Auge des Patienten (10) in der Messposition sichtbar ist.

2. Vorrichtung nach Anspruch 1, wobei die Abschirmwand (210) und das Visierfenster (220) in dem Sinn passiv sind, dass sie zeitlich unveränderliche Lichtdurchlasseigenschaften aufweisen.

3. Vorrichtung nach Anspruch 2, wobei die Abschirmwand (210) das Licht linear oder zirkulär polarisiert und wobei eine Brille (400) oder ein Äquivalent hierfür vorgesehen ist, mit der bzw. dem der Patient (10) in der Messposition ausgerüstet ist und die bzw. das linear bzw. zirkulär polarisierte Gläser (401) enthält, wobei die Gläser (401) und die Abschirmwand (210) in zueinander senkrechten Richtungen geradlinig oder in entgegengesetzten Richtungen zirkulär polarisiert sind.

4. Vorrichtung nach Anspruch 1, wobei die Abschirmwand (210) in dem Sinn aktiv ist, dass sie veränderliche Lichtdurchlasseigenschaften aufweist.

5. Vorrichtung nach Anspruch 4, wobei die Abschirmwand (210) und das Visierfenster (220) gemeinsam einen Flüssigkristallschirm bilden.

6. Vorrichtung nach Anspruch 4, wobei das Visierfenster (220) in dem Sinn inaktiv ist, dass es unveränderliche Lichtdurchlasseigenschaften aufweist, und wobei die Abschirmwand (210) eine aktivierbare Schicht aufweist, die gesteuert werden kann, damit sie eine vollständige Lichtdurchlässigkeit oder Lichtundurchlässigkeit aufweist.

7. Vorrichtung nach einem der vorhergehenden Ansprüche, wobei ein Anzeigeschirm (300) vorgesehen ist, der dafür ausgelegt ist, ein Bild des Patienten (10) in der Messposition, das durch das Erfassungsmittel (110) erfasst wird, anzuzeigen.

8. Vorrichtung nach einem der vorhergehenden Ansprüche, wobei eine Datenverarbeitungseinheit (140) vorgesehen ist, die dafür ausgelegt ist, ein Bild des Patienten (10) in der Messposition, das durch das Erfassungsmittel (110) erfasst wird, zu verarbeiten und daraus das führende Auge des Patienten (10) abzuleiten.

9. Vorrichtung nach einem der vorhergehenden Ansprüche, wobei die Maske (200) einen Rahmen (240) umfasst, der mit wenigstens einem Bedienungsgriff (245) ausgerüstet ist und von dem Patienten (10) getragen werden kann.

10. Verfahren zum Bestimmen des führenden Auges eines Patienten (10) mit Hilfe einer Vorrichtung, die ein Ziel (120), ein Mittel (110) zum Erfassen von Bildern und eine Maske (200), die eine Abschirmwand (210) aufweist, die eine durch ein Visierfenster (220) gebildete Öffnung besitzt, umfasst, **dadurch gekennzeichnet, dass** es Folgendes umfasst:
- einen Schritt des Positionierens des Patienten (10) und der Maske (200) in dem Feld des Mittels (110) zum Erfassen von Bildern, derart, dass sich die Maske (200) zwischen dem Ziel (120) und den beiden Augen des Patienten (10) befindet,
- einen Schritt des Anvisierens des Ziels (120) durch den Patienten (10) durch das Visierfenster (220),
- einen Schritt des Erfassens durch das Erfassungsmittel (110) eines Bildes des Patienten (10), der das Ziel (120) anvisiert, auf dem wenigstens ein Teil des Gesichts des Patienten (10), der ausreicht, um zu ermöglichen, aus dem erfassten Bild abzuleiten, welches der beiden Augen des Patienten sein führendes Auge ist, und ein Teil der Maske (200) erscheinen, und
- einen Schritt des Ableitens des führenden Auges des Patienten (10) als Funktion der Position der Maske (200) in Bezug auf das Gesicht des Patienten (10) auf dem erfassten Bild.

11. Verfahren nach Anspruch 10, wobei der Ableitungsschritt darin besteht, das erfasste Bild auf einem Anzeigeschirm (300) anzuzeigen.

12. Verfahren nach Anspruch 10, wobei in dem Ableitungsschritt das erfasste Bild verarbeitet wird, um einerseits die Positionen der beiden Augen des Patienten (10) und andererseits die Position des Visierfensters (220) in Bezug auf die beiden Augen des Patienten (10) zu berechnen.

13. Verfahren nach Anspruch 10, wobei dann, wenn der Patient (10) mit einer Brille (400) ausgerüstet ist, im Ableitungsschritt das erfasste Bild verarbeitet wird, um einerseits die Positionen der beiden Einfassungen (402) der Brille (400) und andererseits die Position des Visierfensters (220) in Bezug auf die beiden Einfassungen (402) zu berechnen.

14. Verfahren nach Anspruch 10, wobei dann, wenn die Maske (200) wenigstens ein sichtbares Bezugssystem (250) aufweist, das erfasste Bild verarbeitet wird, um die Position des Gesichts des Patienten (10) in Bezug auf das sichtbare Bezugssystem (250) zu berechnen.

15. Verfahren nach einem der Ansprüche 10 bis 14, wobei die Erfassung des Bildes manuell gesteuert wird.

16. Verfahren nach einem der Ansprüche 10 bis 14, wobei die Erfassung des Bildes ununterbrochen in regelmäßigen Intervallen erfolgt und wobei im Ableitungsschritt vorgesehen ist, eines der erfassten Bilder auszuwählen.

17. Verfahren nach Anspruch 14, wobei das Bild ausgewählt wird, wenn die Position des Gesichts des Patienten (10) in Bezug auf die Maske (200) stabil ist.

18. Verfahren nach einem der Ansprüche 10 und 17, wobei ein Schritt des Anzeigens des erfassten Bildes vorgesehen ist.

## Claims

1. A device for determining the dominant eye of a patient (10), **characterized in that** it comprises:
- a target (120) visible by the patient (10) in a measuring position;
- acquisition means (110) for acquiring an image of the face of the patient (10) in the measuring position; and
- a mask (200) to be positioned between, on the one hand, the face of the patient (10) in the measuring position, and on the other hand, the target (120) and the acquisition means (110), comprising:
a) an obstructing panel (210):
a1) that has dimensions such that it is capable of being interposed between the target (120) and the two eyes of the patient (10) in the measuring position;
a2) that is able to transmit only at most some of the light propagating from the target (120) toward the patient (10) in order to hide the target (120) from the patient (10) in the measuring position; and
a3) that is able to transmit at least some of the light propagating from the patient (10) toward the acquisition means (110) in order to allow the acquisition means (110) to acquire an image of the face of the patient (10) in the measuring position, said image being enough to deduce which of the two eyes of the patient is his dominant eye ; and
b) a sighting window (220) used by the patient (10) to sight the target (120):
b1) that is located in the obstructing panel (210);
b2) that is able to transmit the light propagating from the target (120) toward the patient (10), in order to allow the patient (10) to sight the target (120); and
b3) that has dimensions such that the target (120) is visible only by a single eye of the patient (10) in the measuring position.

2. The device as claimed in claim 1, in which the obstructing panel (210) and the sighting window (220) are passive in the sense that they have light transmission properties that are invariable in time.

3. The device as claimed in claim 2, in which the obstructing panel (210) polarizes light linearly or circularly, and in which the patient (10) is equipped with a pair of spectacles (400) or equivalent which comprises lenses (401) that are polarized linearly or circularly, respectively, the lenses (401) and the obstructing panel (210) being rectilinearly polarized in orthogonal directions or circularly polarized in opposite directions.

4. The device as claimed in claim 1, in which the obstructing panel (210) is active in the sense that it has variable light transmission properties.

5. The device as claimed in claim 4, in which the obstructing panel (210) and the sighting window (220) together form a liquid-crystal screen.

6. The device as claimed in claim 4, in which the sighting window (220) is inactive in the sense that it has invariable light transmission properties, and in which the obstructing panel (210) comprises an activatable layer controlled to exhibit complete opacity or transparency.

7. The device as claimed in one of the preceding claims, in which a display screen (300) is provided, said display screen (300) being suitable for displaying an image of the patient (10) in the measuring position, said image being acquired by the acquisition means (110).

8. The device as claimed in one of the preceding claims, in which an information processing unit (140) is provided, said information processing unit (140) being capable of processing an image of the patient (10) in the measuring position and of deducing therefrom the dominant eye of the patient (10), said image being acquired by the acquisition means (110).

9. The device as claimed in one of the preceding claims, in which the mask (200) comprises a chassis (240) equipped with at least one handle (245), and is portable by the patient (10).

10. A method for determining the dominant eye of a patient (10) using a device comprising a target (120), acquisition means (110) for acquiring images, and a mask (200) comprising an obstructing panel (210) apertured with a sighting window (220), **characterized in that** it comprises:
- a step in which the patient (10) and the mask (200) are positioned in the field of the means (110) for acquiring images, in such a way that the mask (200) is located between the target (120) and the two eyes of the patient (10);
- a step in which the patient (10) sights the target (120) through the sighting window (220);
- a step in which the acquisition means (110) acquires an image of the patient (10) sighting the target (120), in which image at least part of the face of the patient (10) and of the mask (200) appear, said image being enough to deduce therefrom which of the two eyes of the patient is his dominant eye; and
- a step in which the dominant eye of the patient (10) is deduced depending on the position of the mask (200) relative to the face of the patient (10) in the acquired image.

11. The method as claimed in claim 10, in which the deduction step consists in displaying the acquired image on a display screen (300).

12. The method as claimed in claim 10, in which, in the deduction step, the acquired image is processed in order to calculate, on the one hand, the positions of the two eyes of the patient (10), and on the other hand, the position of the sighting window (220) relative to the two eyes of the patient (10).

13. The method as claimed in claim 10, in which, the patient (10) being equipped with a pair of spectacles (400), in the deduction step, the acquired image is processed in order to calculate, on the one hand, the positions of the two rims (402) of the pair of spectacles (400), and on the other hand, the position of the sighting window (220) relative to the two rims (402).

14. The method as claimed in claim 10, in which, the mask (200) comprising at least one visible reference mark (250), the acquired image is processed in order to calculate the position of the face of the patient (10) relative to the visible reference mark (250).

15. The method as claimed in one of claims 10 to 14, in which the image acquisition is manually controlled.

16. The method as claimed in one of claims 10 to 14, in which the image acquisition is carried out continuously or at regular intervals, and in which, in the deduction step, provision is made for one of said acquired images to be selected.

17. The method as claimed in claim 14, in which the image is selected when the position of the face of the patient (10) relative to the mask (200) is stabilized.

18. The method as claimed in either one of claims 10 and 17, in which a step of displaying the acquired image is provided.
